# EUROPÄISCHE PATENTSCHRIFT

(11) **EP 3 492 131 B1**
(45) Veröffentlichungstag und Bekanntmachung des Hinweises auf die Patenterteilung: **29.12.2021**
(21) Anmeldenummer: 18000922.7
(22) Anmeldetag: 27.11.2018
(51) Int. Cl.: A61M 16/00, A61M 16/04, A61M 16/12, A61M 16/14, A61M 16/16, A61M 16/20

(54) **SYSTEM ZUR ATEMGASVERSORGUNG**
SYSTEM FOR RESPIRATION GAS SUPPLY
SYSTÈME D'ALIMENTATION EN GAZ DE RESPIRATION

(30) Priorität: 30.11.2017 DE 102017011088
(43) Veröffentlichungstag der Anmeldung: 05.06.2019
(60) Teilanmeldung: 21020479.8
(73) Patentinhaber: Löwenstein Medical Technology S.A., 2557 Luxembourg (LU)
(72) Erfinder: SCHWAIBOLD, Matthias, 76228 Karlsruhe (DE); SCHEERER, Mario, 76530 Baden-Baden (DE); SCHRÖTER, Christof, 76307 Karlsbad (DE)
(74) Vertreter: Marx, Thomas

(56) Entgegenhaltungen:
- WO-A1-2009/146484
- WO-A1-2011/068418
- WO-A1-2016/157105
- US-A1- 2009 151 719
- US-A1- 2013 133 656

## Beschreibung

Die Erfindung betrifft ein System zur Atemgasversorgung umfassend eine Atemgasquelle, eine Steuereinheit, ein Drucksteuermodul und ein Flusssteuermodul, wobei ebenfalls eine Drucksensoreinrichtung und eine Flusssensoreinrichtung und eine Anwenderschnittstelle zur Vorgabe von Parametern der Atemgasversorgung oder zum Austausch von Daten vorgesehen sind. Die Steuereinheit aktiviert alternierend das Drucksteuermodul und das Flusssteuermodul, wobei das Drucksteuermodul die Atemgasquelle zur Vorgabe eines Atemgasdrucks ansteuert und wobei das Flusssteuermodul die Atemgasquelle zur Vorgabe eines Atemgasflusses ansteuert.

### Druckunterstützte und Druckkontrollierte Beatmung

Bei der druckunterstützten Beatmung wird der Atemantrieb des Patienten - mehr oder weniger - berücksichtigt. Sie ist im Gegensatz zur volumenkontrollierten Beatmung schonender, da hier keine hohen Spitzendrücke entstehen können. Wenn der Patient einatmet wird der Atmungsvorgang unterstützt und die Atemarbeit dadurch erleichtert. Gekennzeichnet ist die druckunterstützten Beatmung durch ein unteres Druckniveau und ein oberes Druckniveau. Diese beiden Drücke sind variabel einstellbar. Aus der Menge an Luft die bis Erreichen des oberen Druckes abgegeben wurde, ergibt sich das Atemzugvolumen. Hat der Patient längere Atempausen kann er, mittels einer Backup/Hintergrund-Frequenz, kontrolliert zwischenbeatmet werden. Die Triggerung der Inspiration und Exspiration kann flussgesteuert oder druckgesteuert oder zeitgesteuert erfolgen

Bei einer CPAP-Therapie wird die Spontanatmung eines Patienten mit einem kontinuierlichen Überdruck unterstützt, um Atemstillstände zu vermeiden.

### Volumenkontrollierte Beatmung

Bei dieser Beatmungsform wird dem Patienten, innerhalb einer vorgegebenen Inspirationszeit, ein festgelegtes Atemvolumen appliziert. Das Atemminutenvolumen errechnet sich aus Atemzugvolumen und der Atemfrequenz. Bei dieser Beatmungsform können hohe Spitzendrücke entstehen, da das Beatmungsgerät in erster Linie versucht das eingestellte Volumen zu applizieren. Um dieses Risiko zu verhindern, muss ein Maximaldruck als Alarmgrenze hinterlegt werden.

Mischformen der vorgenannten Beatmungsmodi versuchen die "die Vorteile" der beiden Beatmungsformen (Volumen- und Druckbeatmung) vereinen.

Es ist noch kein System zur Atemgasversorgung bzw. noch keine Atemgasquelle bekannt, die einen hohen Fluss vorgibt und alternierend dazu eine druckunterstützte oder druckkontrollierte Beatmung.

Ein gattungsgemäßer Apparat zur Versorgung mit Atemgas ist in der US 2013/133656 A1 gezeigt. Hierbei wird ein System gezeigt, welches je nach Schlaf- oder Wachphase des Patienten einen Atemfluss oder ein Atemgasdruck vorgibt. Das Atemgas wird zudem angefeuchtet, gegebenenfalls erwärmt und optional mit Sauerstoff versetzt.

Der US 2009/0151719 A1 ist ebenfalls ein System zu entnehmen, welches je nach Tag- oder Nachtzeit zwischen verschiedenen Modi umschaltet. Das System kann optional auch eine Sauerstoffbeimischung umfassen.

Die WO 2011/0684148 A1 unterscheidet zwischen einem Modus, welcher einen Atemgasfluss vorgibt und einem Modus, bei welchem der Atemgasdruck vorgegeben wird. Dabei wird besonders während der Vorgabe des Atemgasflusses das Atemgas angefeuchtet und erwärmt.

Eine effiziente und flexible Methode, dass Atemgas anzufeuchten und zu erwärmen wird in der WO 2009/146484 A1 beschrieben. Optional wird auch beschrieben, dass dem Atemgas Sauerstoff beigemischt wird.

Eine Verbindung einer High-Flow-Therapie und einer CPAP-Therapie in einem Gerät wird durch die WO 2016/157105 A1 beschrieben. Insbesondere während der High-Flow-Therapie wird das Atemgas angefeuchtet und erwärmt sowie Sauerstoff beigemischt.

Die Erfindung ist im unabhänigen Anspruch definiert. Die Erfindung betrifft daher ein System zur Atemgasversorgung umfassend eine Atemgasquelle, eine Steuereinheit, einen Speicher, ein Drucksteuermodul und ein Flusssteuermodul, eine Drucksensoreinrichtung und eine Flusssensoreinrichtung, einen Gasmischer zum Mischen von Umgebungsluft und Sauerstoff (O2) und eine Anwenderschnittstelle zur Vorgabe von Parametern der Atemgasversorgung oder zum Austausch von Daten, mit einem Atemgasschlauch und einem Patienteninterface, wobei das System zusätzlich zumindest einen eine Sauerstoffquelle zur Sauerstoffbeimischung und einen Anfeuchter und eine Heizung zur Erwärmung und Anfeuchtung des Atemgases aufweist. Dabei ist das System auch dadurch gekennzeichnet, dass die Steuereinheit bei Aktivierung des Flusssteuermoduls 6 zusätzlich den Anfeuchter 13 und die Heizung 16 und die Sauerstoffquelle aktiviert. Die Steuereinheit 3 wird derart konfiguriert, dass sie die Atemgasquelle 2 tagsüber zur Vorgabe eines Atemgasflusses ansteuert und nachts zur Vorgabe zumindest eines Atemgasdruckniveaus, welches in Abhängigkeit von Signalen 20 der Drucksensoreinrichtung 7 und/oder der Flusssensoreinrichtung 8 moduliert wird, wenn die Signale 20 indikativ sind für periodische Atmung oder Atemaussetzer, und/oder die Steuereinheit 3 aktiviert nach Vorgabe eines Anwenders über die Anwenderschnittstelle das Drucksteuermodul 4 oder das Flusssteuermodul 6, wobei das Drucksteuermodul 4 die Atemgasquelle 2 zur Vorgabe eines Atemgasdrucks ansteuert und wobei das Flusssteuermodul 6 die Atemgasquelle zur Vorgabe eines Atemgasflusses ansteuert. Die Steuereinheit 3 ist dabei derart konfiguriert, dass sie die Sauerstoffbeimischung so steuert, dass in Phasen der Inspiration dem Patienten mehr Sauerstoff zur Verfügung gestellt wird als zur Exspiration.

Das System ist alternativ oder ergänzend dadurch gekennzeichnet, dass die Steuereinheit bei Aktivierung des Flusssteuermoduls 6 zusätzlich den Anfeuchter 13 und die Heizung 16 und die Sauerstoffquelle 14 zur Konditionierung des Atemgases aktiviert.

Das System ist alternativ oder ergänzend dadurch gekennzeichnet, dass das Flusssteuermodul 6 die Atemgasquelle 2 zur Vorgabe eines Atemgasflusses im Bereich von 0 - 90 l/min, bevorzugt 1 - 80 l/min, besonders bevorzugt 2 - 60 l/min ansteuert.

Das System ist alternativ oder ergänzend dadurch gekennzeichnet, dass das Drucksteuermodul 4 die Atemgasquelle 2 zur Vorgabe eines Atemgasdruckes im Bereich von 0 - 90 mbar, bevorzugt 1 - 80 mbar, besonders bevorzugt 2 - 60 mbar ansteuert.

Das System ist alternativ oder ergänzend dadurch gekennzeichnet, dass das Drucksteuermodul 4 die Atemgasquelle zur Vorgabe eines niedrigen exspiratorischen Druckes und eines höheren inspiratorischen Druckes ansteuert.

Das System ist alternativ oder ergänzend dadurch gekennzeichnet, dass das Drucksteuermodul 4 die Atemgasquelle zur Vorgabe eines konstanten Druckes ansteuert.

Das System ist alternativ oder ergänzend dadurch gekennzeichnet, dass das Drucksteuermodul die Atemgasquelle alternierend zur Vorgabe eines niedrigen exspiratorischen Druckes und eines höheren inspiratorischen Druckes und eines konstanten Druckes ansteuert.

Das System ist alternativ oder ergänzend dadurch gekennzeichnet, dass das Drucksteuermodul die Atemgasquelle zur Vorgabe eines konstanten inspiratorischen Druckes ansteuert.

Das System ist alternativ oder ergänzend dadurch gekennzeichnet, dass das Drucksteuermodul die Atemgasquelle zur Vorgabe eines inspiratorischen Druckes mit einer vorgebbaren Druckwellenform ansteuert.

Das System ist alternativ oder ergänzend dadurch gekennzeichnet, dass das Drucksteuermodul die Atemgasquelle zur Vorgabe eines inspiratorischen Druckes mit zwei unterschiedlichen inspiratorischen Druckniveaus ansteuert.

Das System ist alternativ oder ergänzend dadurch gekennzeichnet, dass das Drucksteuermodul die Atemgasquelle zur Vorgabe eines konstanten exspiratorischen Druckes ansteuert.

Das System ist alternativ oder ergänzend dadurch gekennzeichnet, dass das Drucksteuermodul die Atemgasquelle zur Vorgabe eines exspiratorischen Druckes mit einer vorgebbaren Druckwellenform ansteuert.

Das System ist alternativ oder ergänzend dadurch gekennzeichnet, dass das Drucksteuermodul die Atemgasquelle zur Vorgabe eines exspiratorischen Druckes mit zwei unterschiedlichen exspiratorischen Druckniveaus ansteuert, wobei der Druck ausgehend von einem niedrigen exspiratorischen Niveau auf ein erhöhtes exspiratorisches Niveau angehoben wird.

Das System ist alternativ oder ergänzend dadurch gekennzeichnet, dass das Drucksteuermodul die Atemgasquelle zur Vorgabe eines exspiratorischen Druckes und eines rampenförmigen Druckanstieges auf ein inspiratorisches Druckniveau ansteuert.

Das System ist alternativ oder ergänzend dadurch gekennzeichnet, dass das Drucksteuermodul die Atemgasquelle zur Vorgabe eines inspiratorischen Druckes und eines rampenförmigen Druckabfalls auf ein exspiratorisches Druckniveau ansteuert.

Das System ist alternativ oder ergänzend dadurch gekennzeichnet, dass die Steuereinheit zur Erkennung von Atmungsbemühungen aus dem Drucksignal und/oder aus dem Flusssignal der Drucksensoreinrichtung und/oder der Flusssensoreinrichtung ausgebildet ist.

Das System ist alternativ oder ergänzend dadurch gekennzeichnet, dass die Atemgasquelle ausgebildet ist als Elektromotor mit Lüfterrad.

Das System ist alternativ oder ergänzend dadurch gekennzeichnet, dass das Patienteninterface als Nasenkanüle oder Flusskanüle oder Maske oder als Tracheostomieanschluss ausgebildet ist.

Das System ist alternativ oder ergänzend dadurch gekennzeichnet, dass bei Aktivierung des Flusssteuermoduls als Patienteninterface eine Nasenkanüle oder Flusskanüle oder als ein Tracheostomieanschluss verwendet ist.

Das System ist alternativ oder ergänzend dadurch gekennzeichnet, dass bei Aktivierung des Flusssteuermoduls auch ein interner oder externer Anfeuchter aktiviert ist.

Das System ist alternativ oder ergänzend dadurch gekennzeichnet, dass die Steuereinheit die Atemgasquelle tagsüber zur Vorgabe eines Atemgasflusses ansteuert und nachts zur Vorgabe eines Atemgasdruckes mit niedrigem inspiratorischem Druck und höherem exspiratorischem Druck.

Das System ist alternativ oder ergänzend dadurch gekennzeichnet, dass die Steuereinheit die Atemgasquelle tagsüber zur Vorgabe eines Atemgasflusses ansteuert und nachts zur Vorgabe zumindest eines Atemgasdruckniveaus, welches in Abhängigkeit von Signalen der Drucksensoreinrichtung und/oder der Flusssensoreinrichtung moduliert wird, wenn die Signale indikativ sind für periodische Atmung oder Atemaussetzer.

Das System ist alternativ oder ergänzend dadurch gekennzeichnet, dass die Steuereinheit die Atemgasquelle tagsüber zur Vorgabe eines Atemgasflusses ansteuert und nachts zur Vorgabe zumindest eines Atemgasdruckniveaus, welches in Abhängigkeit von Signalen der Drucksensoreinrichtung und/oder der Flusssensoreinrichtung moduliert wird, wenn die Signale indikativ sind für periodische Atmung oder Atemaussetzer.

Das System ist alternativ oder ergänzend dadurch gekennzeichnet, dass die Steuereinheit 3 die Atemgasquelle 2 zur Vorgabe eines vorgebbaren Atemgasflusses ansteuert und in Abhängigkeit von Signalen 20 der Drucksensoreinrichtung 7 und/oder der Flusssensoreinrichtung 8 den Druck erhöht, bis der vorgegebene Atemgasfluss erreicht ist.

Das System ist alternativ oder ergänzend dadurch gekennzeichnet, dass die Steuereinheit 3 die Atemgasquelle 2 zur Vorgabe eines vorgebbaren Atemgasflusses ansteuert, wobei die Flusssensoreinrichtung 8 den Atemgasfluss überwacht, wobei die Steuereinheit anhand der Signale der Flusssensoreinrichtung 8 prüft, ob der vorgegebene Atemgasfluss erreicht ist, wobei die Drucksensoreinrichtung den Atemgasdruck überwacht, wobei die Steuereinheit anhand der Signale der Drucksensoreinrichtung prüft, ob ein vorgegebener maximale Atemgasdruck erreicht ist und die Steuereinheit den maximalen Atemgasdruck nicht weiter erhöht, wenn der vorgegebene Atemgasfluss nicht erreicht ist.

Das System ist alternativ oder ergänzend dadurch gekennzeichnet, dass die Steuereinheit das Flusssteuermodul 6 aktiviert und dabei versucht, den eingestellten Fluss zu erzeugen und dabei auch den dazu benötigten Druck bestimmt, stößt dieser an eine vorgegebene Schwelle für einen Zeitraum von, typischerweise ca. 0,001 - 10 Sekunden, so schaltet die Steuereinheit auf Drucksteuerung 4 und hält einen vorgegebenen maximal erlaubten Druck, auch wenn der Fluss unter der eingestellten Vorgabe liegt, wenn der eingestellte Fluss wieder erreicht oder überschritten wird, für einen Zeitraum von, typischerweise ca. 0,001 - 10 Sekunden, dann schaltet die Steuereinheit wieder zurück auf die Fluss-Steuerung 6.

Das System dient auch zum Mischen von Umgebungsluft (unter Fluss) und Sauerstoff (O2).

Die Menge des zugeführten Atemgases wird über einen Flussmesser (1-90 l/min) geregelt. Zusätzlich wird über einen Sauerstoffmischer die Applikation der gewünschten Sauerstoffmenge (bis zu 100%) eingestellt.

Das Atemgas wird dem Patient über eine spezielle Nasenbrille oder Kanüle zugeführt, die zur Unterstützung der Spontanatmung dient.

Aufgrund des hohen Gasflusses entsteht ein positiver Atemwegsdruck (PEEP), der von der Höhe des Gasflusses abhängig ist. Durch den hohen Gasfluss wird Kohlendioxid aus dem Nasenrachenraum ausgewaschen.

Durch den hohen Gasfluss wird eine frühere Extubation (Entfernen des Beatmungsschlauchs) ermöglicht.

Der hohe Gasfluss erleichtert die Atemarbeit. Der hohe Gasfluss minimiert die Atemanstrengung. Die Atemfrequenz wird durch hohe Flussraten reduziert.

Die Erfindung betrifft auch eine Atemgasquelle 2 die aufweist, eine Steuereinheit 3, einen Speicher 5, ein Drucksteuermodul 4 und ein Flusssteuermodul 6, eine Drucksensoreinrichtung 7 und eine Flusssensoreinrichtung 8 und eine Anwenderschnittstelle 9 zur Vorgabe von Parametern 10 der Atemgasversorgung oder zum Austausch von Daten, mit einem Atemgasschlauch 11 und einem Patienteninterface 12, wobei das System 1 zusätzlich zumindest einen Anfeuchter 13 und/oder eine Sauerstoffquelle 14 und/oder einen Vernebler 15 und/oder eine Heizung 16 aufweist dadurch gekennzeichnet, dass die Steuereinheit 3 alternierend das Drucksteuermodul 4 oder das Flusssteuermodul 6 aktiviert, wobei das Drucksteuermodul 4 die Atemgasquelle 2 zur Vorgabe eines Atemgasdrucks ansteuert und wobei das Flusssteuermodul 6 die Atemgasquelle zur Vorgabe eines Atemgasflusses ansteuert.

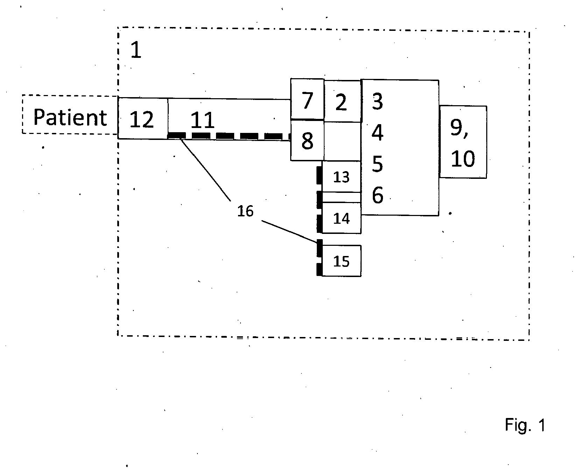

Figur 1 zeigt schematisch das System zur Atemgasversorgung 1 umfassend eine Atemgasquelle 2, eine Steuereinheit 3, ein Drucksteuermodul 4, ein Flusssteuermodul 6 und einen Speicher 5, wobei ebenfalls eine Drucksensoreinrichtung 7 und eine Flusssensoreinrichtung 8 und eine Anwenderschnittstelle 9 zur Vorgabe von Parametern 10 der Atemgasversorgung oder zum Austausch von Daten umfasst sind. Das System weist einen Atemgasschlauch 11 und ein Patienteninterface 12 auf, wobei das System 1 zusätzlich zumindest einen Anfeuchter 13 und/oder eine Sauerstoffquelle 14 und/oder einen Vernebler 15 und/oder eine Heizung 16 aufweist. Die Steuereinheit 3 aktiviert alternierend das Drucksteuermodul 4 und das Flusssteuermodul 6, wobei das Drucksteuermodul 4 die Atemgasquelle 2 zur Vorgabe eines

Atemgasdrucks im Bereich von 0 - 90 mbar ansteuert und wobei das Flusssteuermodul 6 die Atemgasquelle zur Vorgabe eines Atemgasflusses im Bereich von 0 - 90 l/min ansteuert.

Die Steuereinheit 3 ist eingerichtet und ausgebildet die Atemgasquelle 2, unter Verwendung des Drucksteuermoduls 4 oder des Flusssteuermoduls 6, zur automatischen Vorgabe eines Atemgasdruckes und/oder eines Atemgasflusses anzusteuern.

Die Steuereinheit 3 ist eingerichtet und ausgebildet bei Aktivierung des Flusssteuermoduls 4 zusätzlich zumindest zeitweise den Anfeuchter 13 oder den Vernebler und die Heizung 16 zur Erwärmung und Anfeuchtung des Atemgases zu aktivieren.

Bei Aktivierung des Flusssteuermoduls 4 kann die Steuereinheit 3 zusätzlich eine Sauerstoffquelle 14 und/oder einen Vernebler 15 zur Konditionierung des Atemgases aktivieren.

Der Atemgasschlauch 11 und/oder der Anfeuchter 13 und/oder der Vernebler 15 weisen jeweils eine Heizung 16 auf. Alternativ weist zumindest der Atemgasschlauch 11 und/oder der Anfeuchter 13 und/oder der Vernebler 15 eine Heizung 16 auf.

Die Steuereinheit ist zur Erkennung von Atmungsbemühungen aus dem Drucksignal und/oder aus dem Flusssignal der Drucksensoreinrichtung und/oder der Flusssensoreinrichtung eingerichtet und ausgebildet. Die Steuereinheit ist zudem zur Erkennung von periodischer Atmung oder Atemaussetzern aus dem Drucksignal und/oder aus dem Flusssignal der Drucksensoreinrichtung und/oder der Flusssensoreinrichtung eingerichtet und ausgebildet.

Die Anwenderschnittstelle 9 zur Vorgabe von Parametern 10 der Atemgasversorgung oder zum Austausch von Daten ist beispielsweise ausgebildet als ein Touchscreen. Alternativ oder ergänzend können auch ein Display und Eingabetasten vorgesehen sein. Ergänzend weist die Anwenderschnittstelle 9 zumindest eine Schnittstelle (USB, Bluetooth, WLAN...) zur Verbindung mit externen Geräten oder einem Netzwerk auf, um Daten auszutauschen. Vorgegebene Parameter werden im Speicher abrufbar abgelegt.

Die Atemgasquelle 2 ist beispielsweise ausgebildet als Elektromotor mit Lüfterrad oder als Druckgasquelle. Das Patienteninterface 12 ist ausgebildet als Nasenkanüle oder Maske oder als Tracheostomieanschluss ausgebildet ist.

Das System ist ausgebildet und eingerichtet, dass bei Aktivierung des Flusssteuermoduls als Patienteninterface eine Nasenkanüle oder als ein Tracheostomieanschluss verwendet ist und bei

Aktivierung des Flusssteuermoduls auch ein interner oder externer Anfeuchter aktiviert und verwendet ist.

Das System ist beispielsweise ausgebildet und eingerichtet, dass bei Aktivierung des Flusssteuermoduls der interne Befeuchter deaktiviert wird. Stattdessen wird ein externer Anfeuchter verwendet oder aktiviert, der Leistungsstärker ist.

Das System ist beispielsweise ausgebildet und eingerichtet, dass bei Aktivierung des Flusssteuermoduls eine interne Sauerstoffquelle 14 deaktiviert wird. Stattdessen wird eine externe Sauerstoffquelle verwendet.

Das System ist beispielsweise ausgebildet und eingerichtet, dass bei Aktivierung des Flusssteuermoduls eine Sauerstoffbeimischung, über eine interne oder externe Sauerstoffquelle, bis 90 l/min erfolgt.

Das System ist beispielsweise ausgebildet und eingerichtet, dass bei Aktivierung des Flusssteuermoduls die Steuereinheit unter Berücksichtigung der Signale der Fluss- und/oder Drucksensoreinrichtung, eine Sauerstoffquelle zur Sauerstoffbeimischung aktiviert, die Atemphasenabhängig moduliert wird. Beispielsweise ist daran gedacht die Sauerstoffbeimischung so zu steuern, dass in Phasen der Inspiration dem Patienten mehr Sauerstoff zur Verfügung steht als zur Exspiration.

Das System ist beispielsweise ausgebildet und eingerichtet, dass ein Pulsoximeter 17 oder ein Co2-Messgerät 18 adaptiert werden kann, welches dann mit dem System kommuniziert und/oder von ihm mit Energie versorgt wird. Über die Messwerte dieser Geräte kann der Anwender - oder ein automatischer Algorithmus - die richtige Flussrate und die richtige Sauerstoff-Beimischung einstellen. Beispielsweise berücksichtigt die Steuereinheit Messwerte des Pulsoximeters 17 und/oder des Co2-Messgerätes 18 bei der Aktivierung / Steuerung der Sauerstoffquelle und/oder bei der Aktivierung / Steuerung des Flusssteuermoduls 6.

Erfindungsgemäß ist daran gedacht, dass der Anwender über die Anwenderschnittstelle eine Vorgabe an die Steuereinheit 3 eingibt das Drucksteuermodul oder das Flusssteuermodul 6 zu aktivieren. Die Vorgabe kann auch im Speicher abgelegt sein und von der Steuereinheit ausgelesen werden. Erfindungsgemäß ist auch daran gedacht, dass die Steuereinheit 3 selbststätig anhand im Speicher abgelegter Informationen das Modul 4 oder 6 wählt und unter Vorgabe der Parameter (Druckwerte, Flusswerte ...) ansteuert.

Erfindungsgemäß ist auch daran gedacht, dass die Steuereinheit 3 anhand einer Zeitvorgabe das Modul 4 oder 6 wählt und unter Vorgabe der Parameter ansteuert.

Erfindungsgemäß ist auch daran gedacht, dass die Steuereinheit 3 ermittelt welches Patienteninterface adaptiert ist und anhand dieser Information das passende Modul aktiviert. Erfindungsgemäß ist auch daran gedacht, dass die Steuereinheit 3 ermittelt welches Patienteninterface adaptiert ist und abgleicht, ob das Patienteninterface zum aktivierten Modul passt und entweder selbsttätig das passende Modul aktiviert oder einen Alarm aktiviert, um den Anwender auf eine inkompatible Kombination Patienteninterface und Modul hinzuweisen.

Das System erkennt beispielsweise, ob ein Ventil-Beatmungsschlauch angeschlossen ist oder nicht. Das erkennt es daran, ob es in den Steuerleitungen für das Ventil einen Druck aufbauen bzw. messen kann. Ist ein Ventil-Beatmungsschlauch verwendet, aktiviert die Steuereinheit das Flusssteuermodul 6 nicht.

Erfindungsgemäß ist auch daran gedacht, dass die Steuereinheit das Flusssteuermodul 6 aktiviert und dabei bestimmt, welche Druckschwankungen im Schlauch durch die Atmung des Patienten ausgelöst werden. Sind diese größer als ein definierter Schwellenwert, ermittelt die Steuereinheit daraus, dass das Patienteninterface zu dicht oder ungeeignet ist. Es ist also beispielsweise eine Maske oder Tracheostomieanschluss adaptiert und keine Nasenkanüle. Es wird dann ein entsprechender Hinweis oder Alarm ausgegeben. Die Steuereinheit könnte das Flusssteuermodul 6 dann auch deaktivieren.

Erfindungsgemäß ist auch daran gedacht, dass die Steuereinheit das Flusssteuermodul 6 aktiviert und dabei versucht, den eingestellten Fluss zu erzeugen und dabei auch den dazu benötigten Druck bestimmt. Stößt dieser an eine vorgegebene Schwelle für einen Zeitraum von, typischerweise ca. 0,001 - 10 Sekunden, so schaltet die Steuereinheit auf Drucksteuerung 4 und hält einen vorgegebenen maximal erlaubten Druck, auch wenn der Fluss unter der eingestellten Vorgabe liegt. Wenn nun der eingestellte Fluss wieder erreicht oder überschritten wird, für einen Zeitraum von, typischerweise ca. 0,001 - 10 Sekunden, dann schaltet das Gerät wieder zurück auf die Fluss-Steuerung 6.

Erfindungsgemäß ist auch daran gedacht, dass die Steuereinheit das Flusssteuermodul 6 aktiviert und dabei bestimmt, welche Atemphasen vorliegt. Die Steuereinheit erkennt Schwankungen durch die Atmung und versucht, diese kontinuierlich auszugleichen.

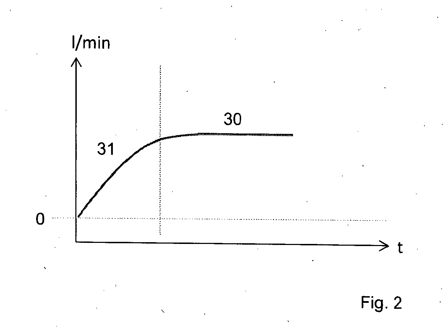

Figur 2 zeigt schematisch einen Flussverlauf 30,31 des Systems zur Atemgasversorgung 1. Die Atemgasquelle 2 wird hier von der Steuereinheit 3 unter Verwendung des Flusssteuermoduls 6 zur Vorgabe eines Atemgasflusses 30 im Bereich von 0 - 90 l/min ansteuert. Man erkennt, dass der Fluss 30 allmählich ansteigt 31 und schließlich auf dem vorgegebenen Wert 30 konstant gehalten wird.

Die Flusssensoreinrichtung 8 ermittelt dabei den aktuellen Fluss und die Steuereinheit gleicht diesen mit dem vorgegebenen Fluss ab und regelt den vorgegebenen Fluss 30 mit hoher Genauigkeit von zumindest +/- 5 % im Flussbereich von 5 bis 70 l/min ein. Die Vorgabe ist im Bereich von 0 - 90 l/min, bevorzugt von 0 - 60 l/min in Schritten von beispielsweise 0,5 l/min möglich. Falls vom Anwender gewünscht ist eine Sauerstoffbeimischung von 0-90 l/min, bevorzugt 1 - 45 l/min möglich. Dazu aktiviert die Steuereinheit die Sauerstoffquelle 14. Zudem aktiviert die Steuereinheit, bei Verwendung des Flusssteuermoduls 6, immer die Heizung 16 und den Anfeuchter 13 oder den Vernebler 15 zur Konditionierung des Atemgases auf eine vorgebbare Temperatur und eine vorgebbare Feuchte. Temperatur und Feuchte liegen typischerweise immer über derjenigen der Umgebungsluft. Die Temperatur ist im Bereich von 30°- 37°C, in beispielsweise 1°C-Schritten einstellbar.

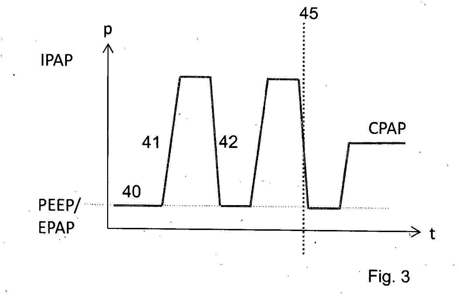

Figur 3 zeigt schematisch einen Druckverlauf 40,41... des Systems zur Atemgasversorgung 1. Die Atemgasquelle 2 wird hier von der Steuereinheit 3 unter Verwendung des Drucksteuermoduls 4 zur Vorgabe eines Atemgasdruckes 40 im Bereich von 0 - 90 mbar ansteuert. Man erkennt, dass der Druck 40 von einem PEEP oder EPAP Niveau ansteigt auf einen IPAP.

Der Übergang vom PEEP oder EPAP Niveau auf den IPAP kann in Form einer vorgebaren Rampe 41 oder Wellenform erfolgen. Ebenso kann der Übergang vom IPAP auf den EPAP in Form einer vorgebaren Rampe 42 oder Wellenform erfolgen. Der EPAP Druck kann konstant sein oder von einem initial geringen EPAP zum Ende der Exspiration ansteigen. Es ist erfindungsgemäß auch denkbar, dass der EPAP zumindest zwei unterschiedliche Druckniveaus umfasst.

Die Flusssensoreinrichtung 8 und/oder die Drucksensoreinrichtung ermittelt dabei den aktuellen Fluss und/oder Druck und die Steuereinheit ermittelt aus diesen Werten die Atemphase des Patienten und berücksichtigt diese zumindest zeitweise bei der Vorgabe der Drücke IPAP und EPAP. Die Steuereinheit gleicht den Druck mit dem vorgegebenen Druck (aus dem Speicher 5) ab und regelt den Druck mit hoher Genauigkeit von zumindest +/- 5 % im Bereich von 2 bis 45 mbar ein.

Figur 3 zeigt zudem, dass auf Anwenderauswahl 45 oder auf ein Ereignis 45 hin die Steuereinheit den Druck auf einem konstanten CPAP Niveau vorgibt. Das Ereignis erkennt die Steuereinheit beispielsweise aus den Signalen der Flusssensoreinrichtung 8 und/oder der Drucksensoreinrichtung als Atemaussetzer oder als periodische Atmung.

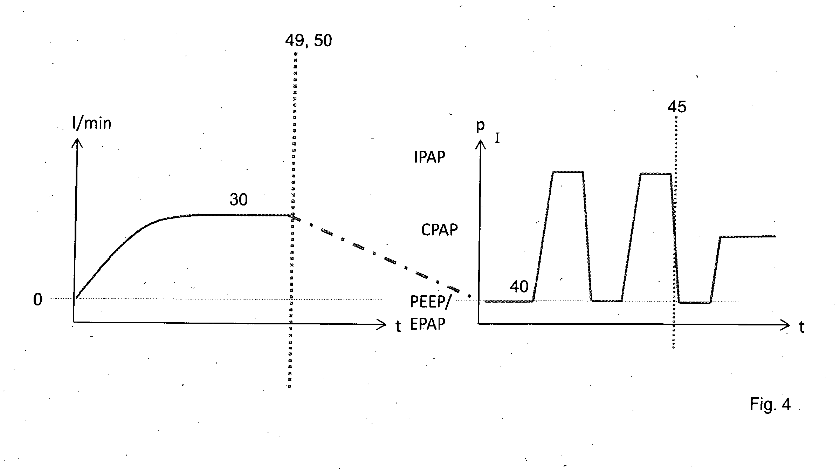

Figur 4 zeigt schematisch einen Flussverlauf 30 des Systems zur Atemgasversorgung 1. Die Atemgasquelle 2 wird hier von der Steuereinheit 3 unter Verwendung des Flusssteuermoduls 6 zur Vorgabe eines Atemgasflusses 30 im Bereich von 0 - 90 l/min angesteuert. Figur 4 zeigt zudem schematisch einen Druckverlauf 40 des Systems zur Atemgasversorgung 1. Die Atemgasquelle 2 wird hier von der Steuereinheit 3 unter Verwendung des Drucksteuermoduls 4 zur Vorgabe eines Atemgasdruckes 40 im Bereich von 0 - 90 mbar auf EPAP oder IPAP oder CPAP-Druck angesteuert. Aus Figur 4 ist zudem ersichtlich, dass die Steuereinheit 3 alternierend das Flusssteuermodul 4 und dann das Drucksteuermodul 4 aktiviert. Der Übergang von der Flusssteuerung zur Drucksteuerung oder von der Drucksteuerung zur Flusssteuerung kann auf Anwenderauswahl 49 hin über die Anwenderschnittstelle 9 erfolgen oder automatisch auf ein Ereignis 50 hin unter Vorgabe der Steuereinheit oder automatisch auf einen vorbestimmen Ablauf hin der von der Steuereinheit aus dem Speicher 5 gelesen wird. Ein solches Ereignis 50 kann der Wechsel der Tageszeit sein. Die Steuereinheit weist dazu eine Uhr auf, die entsprechend vorgebbarer Zeitpunkte vom Tag- auf den Nacht-Modus umschaltet. Die Steuereinheit kann den Wechsel vom Tag- auf den Nacht-Modus auf eine Anwendereingabe hin, einleiten oder aufgrund einer Sensoreinrichtung, die den Schlaf des Anwenders feststellt. Die Steuereinheit 3 kann das Flusssteuermodul aktivieren, wobei diese die Atemgasquelle 2 tagsüber zur Vorgabe eines Atemgasflusses ansteuern. Die Steuereinheit 3 registriert den Wechsel der Tageszeit und aktiviert nachts die Drucksteuerung zur Vorgabe eines Atemgasdruckes mit niedrigem inspiratorischem Druck und höherem exspiratorischem Druck oder auf einem CPAP-Niveau.

Das System nach Fig. 4 ist auch eingerichtet und ausgebildet, dass die Steuereinheit 3 tagsüber das Fluss-Modul 6 aktiviert, wobei das Fluss-Modul 5 die Atemgasquelle 2 zur Vorgabe eines Atemgasflusses ansteuert und nachts zur Vorgabe zumindest eines Atemgasdruckniveaus, welches in Abhängigkeit von Signalen 20 der Drucksensoreinrichtung 7 und/oder der Flusssensoreinrichtung 8 moduliert wird, wenn die Signale 20 indikativ sind für periodische Atmung oder Atemaussetzer. Das Atemgasdruckniveau wird, wenn die Signale 20 indikativ sind für periodische Atmung so moduliert, dass die Druckvorgaben die periodische Atmung dämpfen oder verstärken. Das Atemgasdruckniveau wird, wenn die Signale 20 indikativ sind für Atemaussetzer so moduliert, dass die Druckvorgaben eine höhere Druckdifferenz EPAP - IPAP oder einen CPAP vorgeben.

Das erfindungsgemäße High-Flow System zur Atemgasversorgung baut durch einen Gasmischer einen Frischgasflow von bis zu 90l/min auf, wodurch der Durchfluss höher ist als bei Beatmungs-Geräten. Durch den hohen Fluss strömt das Atemgas kontinuierlich zum Patienten, ohne dass dessen Eigenatmung behindert wird. Zum System gehört ein Gasmischer (Druckluft oder Lüfer und Sauerstoff), ein Atemwegsanfeuchter (der das Atemgas zugleich erwärmt), ggfs. noch ein Reservoirbeutel und ein PEEP-Ventil. Der durch das PEEP-Ventil am Ende der Exspiration erzeugte Druck bleibt auch während der Inspiration erhalten, da während des gesamten Atemzyklus ein hoher Atemgasfluss vorhanden ist. Das System reduziert High-Flow-CPAP die Gefahr von Druckinkonstanzen unter Masken-CPAP.

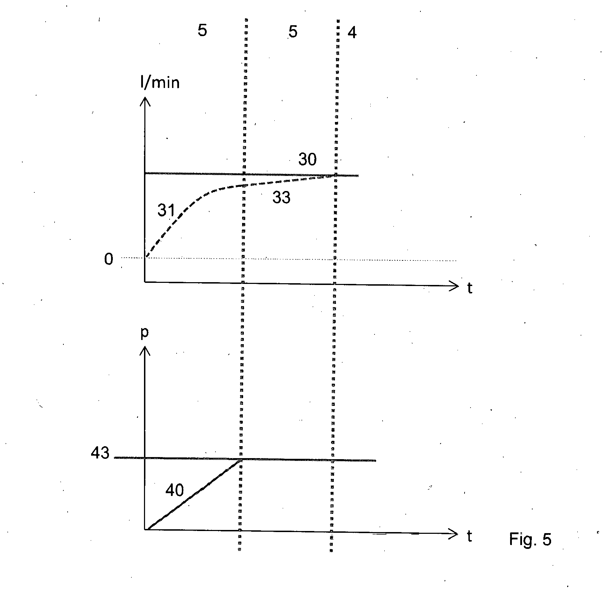

Das System nach Fig. 5 ist auch eingerichtet und ausgebildet, dass die Steuereinheit 3 das Fluss-Modul 6 aktiviert, wobei das Fluss-Modul 6 die Atemgasquelle 2 zur Vorgabe eines vorgebbaren Atemgasflusses 30 ansteuert und in Abhängigkeit von Signalen 20 der Drucksensoreinrichtung 7 und/oder der Flusssensoreinrichtung 8 den Druck 40 erhöht, bis der vorgegebene Atemgasfluss 30 erreicht ist. Das System ist auch eingerichtet, dass die Flusssensoreinrichtung 8 dabei den Atemgasfluss überwacht, wobei das Fluss-Modul 6 anhand der Signale der Flusssensoreinrichtung 8 prüft, ob der vorgegebene Atemgasfluss erreicht ist und wobei die Drucksensoreinrichtung den Atemgasdruck überwacht, wobei das Fluss-Modul 6 anhand der Signale der Drucksensoreinrichtung prüft, ob ein vorgegebener maximaler Atemgasdruck 43 erreicht ist und Fluss-Modul 6 den maximalen Atemgasdruck nicht weiter erhöht, bis der vorgegebene Atemgasfluss 30 erreicht ist. Dabei kann der aktuelle Fluss 33 für einige Sekunden oder Minuten unterhalb der Vorgabe 30 bleiben und sich ggfs. nur langsam erhöhen. Wenn der vorgegebene Atemgasfluss 30 erreicht ist kann eine Umschaltung auf das Flusssteuermodul 4 erfolgen.

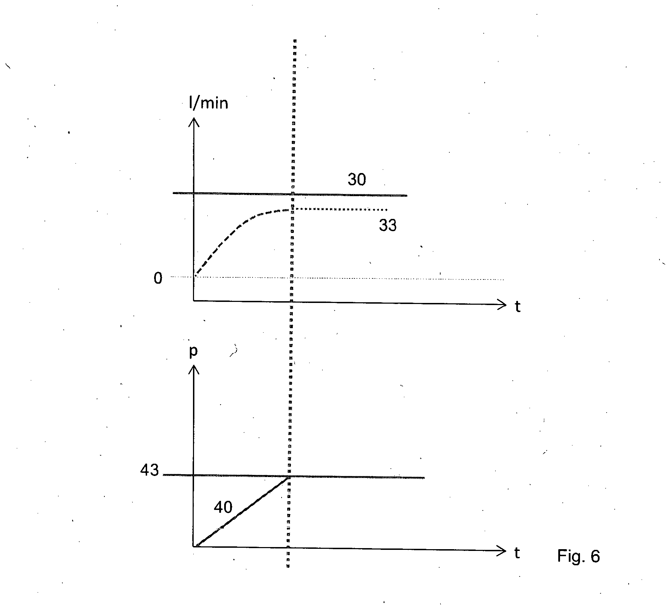

Das System nach Fig. 6 ist auch eingerichtet und ausgebildet, dass die Steuereinheit 3 das Fluss-Modul 6 aktiviert, wobei das Fluss-Modul 6 die Atemgasquelle 2 zur Vorgabe eines vorgebbaren Atemgasflusses 30 ansteuert und in Abhängigkeit von Signalen 20 der Drucksensoreinrichtung 7 und/oder der Flusssensoreinrichtung 8 den Druck 40 erhöht, bis der vorgegebene Atemgasfluss 30 erreicht ist. Das System ist auch eingerichtet, dass die Flusssensoreinrichtung 8 dabei den Atemgasfluss überwacht, wobei das Fluss-Modul 6 oder die Steuereinheit 3 anhand der Signale der Flusssensoreinrichtung 8 prüft, ob der vorgegebene Atemgasfluss erreicht ist und wobei die Drucksensoreinrichtung den Atemgasdruck überwacht, wobei das Fluss-Modul 6 oder die Steuereinheit 3 anhand der Signale der Drucksensoreinrichtung prüft, ob ein vorgegebener maximaler Atemgasdruck 43 erreicht ist und das Fluss-Modul 6 oder die Steuereinheit 3 den maximalen Atemgasdruck nicht weiter erhöht, wenn der vorgegebene Atemgasfluss 30 nicht erreicht ist. Der maximale Atemgasdruck 43 wird auch dann nicht weiter erhöht, wenn der aktuelle Atemgasfluss 33 nicht die Vorgabe 30 erreicht.

## Patentansprüche

1. High-Flow-CPAP-System zur Atemgasversorgung 1 umfassend eine Atemgasquelle 2, eine Steuereinheit 3, einen Speicher 5, ein Drucksteuermodul 4 und ein Flusssteuermodul 6, eine Drucksensoreinrichtung 7 und eine Flusssensoreinrichtung 8, einen Gasmischer zum Mischen von Umgebungsluft und Sauerstoff (O2) und eine Anwenderschnittstelle 9 zur Vorgabe von Parametern 10 der Atemgasversorgung oder zum Austausch von Daten, mit einem Atemgasschlauch 11 und einem Patienteninterface 12, wobei das System 1 zusätzlich zumindest eine Sauerstoffquelle 14 zur Sauerstoffbeimischung und einen Anfeuchter 13 und eine Heizung 16 zur Erwärmung und Anfeuchtung des Atemgases aufweist wobei die Steuereinheit 3 derart konfiguriert wird, dass sie die Atemgasquelle 2 tagsüber zur Vorgabe eines Atemgasflusses ansteuert und nachts zur Vorgabe zumindest eines Atemgasdruckniveaus, welches in Abhängigkeit von Signalen 20 der Drucksensoreinrichtung 7 und/oder der Flusssensoreinrichtung 8 moduliert wird, wenn die Signale 20 indikativ sind für periodische Atmung oder Atemaussetzer, und/oder die Steuereinheit 3 nach Vorgabe eines Anwenders über die Anwenderschnittstelle das Drucksteuermodul 4 oder das Flusssteuermodul 6 aktiviert, wobei das Drucksteuermodul 4 die Atemgasquelle 2 zur Vorgabe eines Atemgasdrucks ansteuert und wobei das Flusssteuermodul 6 die Atemgasquelle zur Vorgabe eines Atemgasflusses ansteuert, wobei das Drucksteuermodul 4 die Atemgasquelle 2 zur Vorgabe eines Atemgasdrucks ansteuert und wobei das Flusssteuermodul 6 die Atemgasquelle zur Vorgabe eines Atemgasflusses ansteuert, **dadurch gekennzeichnet, dass**
die Steuereinheit derart konfiguriert ist, dass sie bei Aktivierung des Flusssteuermoduls 6 zusätzlich den Anfeuchter 13 und
die Heizung 16 und die Sauerstoffquelle 14 aktiviert, sodass die Steuereinheit 3 die Sauerstoffbeimischung so steuert, dass in Phasen der Inspiration dem Patienten mehr Sauerstoff zur Verfügung gestellt wird als zur Exspiration.

2. System nach Anspruch 1 **dadurch gekennzeichnet, dass** das System 1 zusätzlich zumindest einen Anfeuchter 13 und eine Heizung 16 aufweist **dadurch gekennzeichnet, dass** die Steuereinheit 3 alternierend das Drucksteuermodul 4 oder das Flusssteuermodul 6 aktiviert, wobei das Drucksteuermodul 4 die Atemgasquelle 2 zur Vorgabe eines Atemgasdrucks ansteuert und wobei das Flusssteuermodul 6 die Atemgasquelle zur Vorgabe eines Atemgasflusses ansteuert, wobei die Steuereinheit bei Aktivierung des Flusssteuermoduls 6 zusätzlich den Anfeuchter 13 und die Heizung 16 zur Erwärmung und Anfeuchtung des Atemgases aktiviert und bei Aktivierung des Flusssteuermoduls als Patienteninterface eine Nasenkanüle oder Flusskanüle oder ein Tracheostomieanschluss verwendet ist.

3. System nach zumindest einem der vorhergehenden Ansprüche **dadurch gekennzeichnet, dass** das Drucksteuermodul die Atemgasquelle zur Vorgabe eines inspiratorischen Druckes mit einer vorgebbaren Druckwellenform ansteuert.

4. System nach zumindest einem der vorhergehenden Ansprüche **dadurch gekennzeichnet, dass** das Drucksteuermodul die Atemgasquelle zur Vorgabe eines inspiratorischen Druckes mit zwei unterschiedlichen inspiratorischen Druckniveaus ansteuert.

5. System nach zumindest einem der vorhergehenden Ansprüche **dadurch gekennzeichnet, dass** das Drucksteuermodul die Atemgasquelle zur Vorgabe eines exspiratorischen Druckes mit einer vorgebbaren Druckwellenform ansteuert.

6. System nach zumindest einem der vorhergehenden Ansprüche **dadurch gekennzeichnet, dass** das Drucksteuermodul die Atemgasquelle zur Vorgabe eines exspiratorischen Druckes mit zwei unterschiedlichen exspiratorischen Druckniveaus ansteuert, wobei der Druck ausgehend von einem niedrigen exspiratorischen Niveau auf ein erhöhtes exspiratorisches Niveau angehoben wird.

7. System nach zumindest einem der vorhergehenden Ansprüche **dadurch gekennzeichnet, dass** das Drucksteuermodul die Atemgasquelle zur Vorgabe eines exspiratorischen Druckes und eines rampenförmigen Druckanstieges auf ein inspiratorisches Druckniveau ansteuert und dass das Drucksteuermodul die Atemgasquelle zur Vorgabe eines inspiratorischen Druckes und eines rampenförmigen Druckabfalls auf ein exspiratorisches Druckniveau ansteuert.

8. System nach zumindest einem der vorhergehenden Ansprüche **dadurch gekennzeichnet, dass** die Steuereinheit zur Erkennung von Atmungsbemühungen aus dem Drucksignal und/oder aus dem Flusssignal der Drucksensoreinrichtung und/oder der Flusssensoreinrichtung ausgebildet ist.

9. System nach zumindest einem der vorhergehenden Ansprüche **dadurch gekennzeichnet, dass** die Steuereinheit 3 die Atemgasquelle 2 tagsüber zur Vorgabe eines Atemgasflusses ansteuert und nachts zur Vorgabe eines Atemgasdruckes mit niedrigem inspiratorischem Druck und höherem exspiratorischem Druck.

10. System nach zumindest einem der vorhergehenden Ansprüche **dadurch gekennzeichnet, dass** die Steuereinheit 3 die Atemgasquelle 2 tagsüber zur Vorgabe eines Atemgasflusses ansteuert und nachts zur Vorgabe zumindest eines Atemgasdruckniveaus, welches in Abhängigkeit von Signalen 20 der Drucksensoreinrichtung 7 und/oder der Flusssensoreinrichtung 8 moduliert wird, wenn die Signale 20 indikativ sind für periodische Atmung oder Atemaussetzer.

11. System nach zumindest einem der vorhergehenden Ansprüche **dadurch gekennzeichnet, dass** die Steuereinheit 3 die Atemgasquelle 2 zur Vorgabe eines vorgebbaren Atemgasflusses ansteuert und in Abhängigkeit von Signalen 20 der Drucksensoreinrichtung 7 und/oder der Flusssensoreinrichtung 8 den Druck erhöht, bis der vorgegebene Atemgasfluss erreicht ist.

12. System nach zumindest einem der vorhergehenden Ansprüche **dadurch gekennzeichnet, dass** die Steuereinheit 3 die Atemgasquelle 2 zur Vorgabe eines vorgebbaren Atemgasflusses ansteuert, wobei die Flusssensoreinrichtung 8 den Atemgasfluss überwacht, wobei die Steuereinheit anhand der Signale der Flusssensoreinrichtung 8 prüft, ob der vorgegebene Atemgasfluss erreicht ist, wobei die Drucksensoreinrichtung den Atemgasdruck überwacht, wobei die Steuereinheit anhand der Signale der Drucksensoreinrichtung prüft, ob ein vorgegebener maximale Atemgasdruck erreicht ist und die Steuereinheit den maximalen Atemgasdruck nicht weiter erhöht, wenn der vorgegebene Atemgasfluss nicht erreicht ist.

13. System nach zumindest einem der vorhergehenden Ansprüche **dadurch gekennzeichnet, dass** die Steuereinheit das Flusssteuermodul 6 aktiviert und dabei versucht, den eingestellten Fluss zu erzeugen und dabei auch den dazu benötigten Druck bestimmt, stößt dieser an eine vorgegebene Schwelle für einen Zeitraum von, typischerweise ca. 0,001 - 10 Sekunden, so schaltet die Steuereinheit auf Drucksteuerung 4 und hält einen vorgegebenen maximal erlaubten Druck, auch wenn der Fluss unter der eingestellten Vorgabe liegt, wenn der eingestellte Fluss wieder erreicht oder überschritten wird, für einen Zeitraum von, typischerweise ca. 0,001 - 10 Sekunden, dann schaltet die Steuereinheit wieder zurück auf die Fluss-Steuerung 6.

## Claims

1. A high-flow CPAP system for respiration gas supply 1 comprising a respiration gas source 2, a control unit 3, a memory 5, a pressure control module 4 and a flow control module 6, a pressure sensor apparatus 7 and a flow sensor apparatus 8, a gas mixer for mixing ambient air and oxygen (O₂) and a user interface 9 for predetermining parameters 10 of the respiration gas supply or for exchanging data, with a respiration gas tube 11 and a patient interface 12, wherein the system 1 additionally has at least one oxygen source 14 for mixing oxygen and a humidifier 13 and a heater 16 for heating and humidifying the respiration gas, wherein the control unit 3 is configured such that it actuates the respiration gas source 2 during the daytime for predetermining a respiration gas flow and during the night time for predetermining at least one respiration gas pressure level which is modulated as a function of signals 20 of the pressure sensor apparatus 7 and/or the flow sensor apparatus 8 when the signals 20 indicate periodic respiration or interrupted respiration, and/or the control unit 3 activates the pressure control module 4 or the flow control module 6 after predetermination by a user via the user interface, wherein the pressure control module 4 actuates the respiration gas source 2 for predetermining a respiration gas pressure and wherein the flow control module 6 actuates the respiration gas source for predetermining a respiration gas flow, wherein the pressure control module 4 actuates the respiration gas source 2 for predetermining a respiration gas pressure and wherein the flow control module 6 actuates the respiration gas source for predetermining a respiration gas flow, **characterized in that** the control unit is configured such that it additionally activates the humidifier 13 and the heater 16 and the oxygen source 14 when the flow control module 6 is activated, such that the control unit 3 controls the oxygen mixture so that more oxygen is available to the patient in the inspiration phases than for the expiration.

2. The system according to claim 1, **characterized in that** the system 1 additionally has at least one humidifier 13 and a heater 16, **characterized in that** the control unit 3 alternately activates the pressure control module 4 or the flow control module 6, wherein the pressure control module 4 actuates the respiration gas source 2 for predetermining a respiration gas pressure and wherein the flow control module 6 actuates the respiration gas source for predetermining a respiration gas flow, wherein when the flow control module 6 is activated the control unit additionally activates the humidifier 13 and the heater 16 for heating and humidifying the respiration gas, and when the flow control module is activated a nasal cannula or flow cannula or a tracheostomy port is used as a patient interface.

3. The system according to at least one of the preceding claims, **characterized in that** the pressure control module actuates the respiration gas source for predetermining an inspiratory pressure with a predeterminable pressure waveform.

4. The system according to at least one of the preceding claims, **characterized in that** the pressure control module actuates the respiration gas source for predetermining an inspiratory pressure with two different inspiratory pressure levels.

5. The system according to at least one of the preceding claims, **characterized in that** the pressure control module actuates the respiration gas source for predetermining an expiratory pressure with a predeterminable pressure waveform.

6. The system according to at least one of the preceding claims, **characterized in that** the pressure control module actuates the respiration gas source for predetermining an expiratory pressure with two different expiratory pressure levels, wherein the pressure is raised from a low expiratory level to an elevated expiratory level.

7. The system according to at least one of the preceding claims, **characterized in that** the pressure control module actuates the respiration gas source for predetermining an expiratory pressure and a ramped pressure increase to an inspiratory pressure level and **in that** the pressure control module actuates the respiration gas source for predetermining an inspiratory pressure and a ramped pressure drop to an expiratory pressure level.

8. The system according to at least one of the preceding claims, **characterized in that** the control unit is configured for identifying respiratory efforts from the pressure signal and/or from the flow signal of the pressure sensor apparatus and/or the flow sensor apparatus.

9. The system according to at least one of the preceding claims, **characterized in that** the control unit 3 actuates the respiration gas source 2 during the daytime for predetermining a respiration gas flow and during the night time for predetermining a respiration gas pressure with a low inspiratory pressure and higher expiratory pressure.

10. The system according to at least one of the preceding claims, **characterized in that** the control unit 3 actuates the respiration gas source 2 during the daytime for predetermining a respiration gas flow and during the night time for predetermining at least one respiration gas pressure level, which is modulated as a function of signals 20 of the pressure sensor apparatus 7 and/or the flow sensor apparatus 8 when the signals 20 indicate periodic respiration or interrupted respiration.

11. The system according to at least one of the preceding claims, **characterized in that** the control unit 3 actuates the respiration gas source 2 for predetermining a predeterminable respiration gas flow and increases the pressure as a function of signals 20 of the pressure sensor apparatus 7 and/or the flow sensor apparatus 8 until the predetermined respiration gas flow is reached.

12. The system according to at least one of the preceding claims, **characterized in that** the control unit 3 actuates the respiration gas source 2 for predetermining a predeterminable respiration gas flow, wherein the flow sensor apparatus 8 monitors the respiration gas flow, wherein using the signals of the flow sensor apparatus 8 the control unit checks whether the predetermined respiration gas flow is reached, wherein the pressure sensor apparatus monitors the respiration gas pressure, wherein using the signals of the pressure sensor apparatus the control unit checks whether a predetermined maximum respiration gas pressure is reached and the control unit does not increase the maximum respiration gas pressure further if the predetermined respiration gas flow is not reached.

13. The system according to at least one of the preceding claims, **characterized in that** the control unit activates the flow control module 6 and thereby attempts to generate the set flow and at the same time also determines the pressure required therefor, and if this pressure reaches a predetermined threshold for a time period of typically ca. 0.001 - 10 seconds, the control unit switches to pressure control 4 and maintains a predetermined maximum permitted pressure even if the flow is below the set predetermined value, and if the set flow is reached or exceeded again for a time period of typically ca. 0.001 - 10 seconds, then the control unit switches back again to the flow control 6.

## Revendications

1. Système CPAP High-Flow pour l'alimentation en gaz de respiration 1, comportant une source de gaz de respiration 2, une unité de commande 3, une mémoire 5, un module de commande de pression 4 et un module de commande d'écoulement 6, un dispositif de capteur de pression 7 et un dispositif de capteur d'écoulement 8, un mélangeur de gaz destiné à mélanger de l'air ambiant et de l'oxygène (O₂) et une interface d'utilisateur 9 pour la spécification de paramètres 10 de l'alimentation en gaz de respiration ou pour l'échange de données, avec un tube de gaz de respiration 11 et une interface de patient 12, dans lequel le système 1 présente en outre au moins une source d'oxygène 14 destinée à ajouter de l'oxygène et un humidificateur 13 et un chauffage 16 destiné à chauffer et humidifier le gaz de respiration, dans lequel l'unité de commande 3 est configurée de manière à actionner la source de gaz de respiration 2 pendant la journée pour la spécification d'un écoulement de gaz de respiration et pendant la nuit pour la spécification d'au moins un niveau de pression de gaz de respiration, lequel est modulé en fonction de signaux 20 du dispositif de capteur de pression 7 et/ou du dispositif de capteur d'écoulement 8, lorsque les signaux 20 indiquent une respiration périodique ou des arrêts de respiration, et/ou l'unité de commande 3 active le module de commande de pression 4 ou le module de commande d'écoulement 6 selon les spécifications d'un utilisateur par le biais de l'interface d'utilisateur, dans lequel le module de commande de pression 4 actionne la source de gaz de respiration 2 pour la spécification d'une pression de gaz de respiration et dans lequel le module de commande d'écoulement 6 actionne la source de gaz de respiration pour la spécification d'un écoulement de gaz de respiration, dans lequel le module de commande de pression 4 actionne la source de gaz de respiration 2 pour la spécification d'une pression de gaz de respiration et dans lequel le module de commande d'écoulement 6 actionne la source de gaz de respiration pour la spécification d'un écoulement de gaz de respiration, **caractérisé en ce que** l'unité de commande est configurée de manière à activer également l'humidificateur 13 et le chauffage 16 et la source d'oxygène 14 lors de l'activation du module de commande d'écoulement 6, de telle façon que l'unité de commande 3 commande l'ajout d'oxygène de manière à fournir une plus grande quantité d'oxygène au patient dans des phases de l'inspiration que pour l'expiration.

2. Système selon la revendication 1, **caractérisé en ce que** le système 1 présente en outre au moins un humidificateur 13 et un chauffage 16, **caractérisé en ce que** l'unité de commande 3 active alternativement le module de commande de pression 4 ou le module de commande d'écoulement 6, dans lequel le module de commande de pression 4 actionne la source de gaz de respiration 2 pour la spécification d'une pression de gaz de respiration et dans lequel le module de commande d'écoulement 6 actionne la source de gaz de respiration pour la spécification d'un écoulement de gaz de respiration, dans lequel, lors de l'activation du module de commande d'écoulement 6, l'unité de commande active également l'humidificateur 13 et le chauffage 16 pour chauffer et humidifier le gaz de respiration et, lors de l'activation du module de commande d'écoulement, une canule nasale ou une canule d'écoulement ou un raccord de trachéostomie est utilisé en tant qu'interface de patient.

3. Système selon l'une au moins des revendications précédentes, **caractérisé en ce que** le module de commande de pression actionne la source de gaz de respiration pour la spécification d'une pression inspiratoire avec une forme d'onde de pression prédéfinissable.

4. Système selon l'une des revendications précédentes, **caractérisé en ce que** le module de commande de pression actionne la source de gaz de respiration pour la spécification d'une pression inspiratoire avec deux niveaux de pression inspiratoire différents.

5. Système selon l'une au moins des revendications précédentes, **caractérisé en ce que** le module de commande de pression actionne la source de gaz de respiration pour la spécification d'une pression expiratoire avec une forme d'onde de pression prédéfinissable.

6. Système selon l'une au moins des revendications précédentes, **caractérisé en ce que** le module de commande de pression actionne la source de gaz de respiration pour la spécification d'une pression expiratoire avec deux niveaux de pression expiratoire différents, dans lequel la pression est augmentée à un niveau expiratoire élevé à partir d'un niveau expiratoire faible.

7. Système selon l'une au moins des revendications précédentes, **caractérisé en ce que** le module de commande de pression actionne la source de gaz de respiration pour la spécification d'une pression expiratoire et d'une augmentation de pression en forme de rampe à un niveau de pression inspiratoire, et **en ce que** le module de commande de pression actionne la source de gaz de respiration pour la spécification d'une pression inspiratoire et d'une réduction de pression en forme de rampe à un niveau de pression expiratoire.

8. Système selon l'une au moins des revendications précédentes, **caractérisé en ce que** l'unité de commande est conçue pour détecter des efforts de respiration à partir du signal de pression et/ou à partir du signal d'écoulement du dispositif de capteur de pression et/ou du dispositif de capteur d'écoulement.

9. Système selon l'une au moins des revendications précédentes, **caractérisé en ce que** l'unité de commande 3 actionne la source de gaz de respiration 2 pendant la journée pour la spécification d'un écoulement de gaz de respiration et pendant la nuit pour la spécification d'une pression de gaz de respiration avec une pression inspiratoire faible et une pression expiratoire plus élevée.

10. Système selon l'une au moins des revendications précédentes, **caractérisé en ce que** l'unité de commande 3 actionne la source de gaz de respiration 2 pendant la journée pour la spécification d'une écoulement de gaz de respiration et pendant la nuit pour la spécification d'au moins un niveau de pression de gaz de respiration, lequel est modulé en fonction de signaux 20 du dispositif de capteur de pression 7 et/ou du dispositif de capteur d'écoulement 8, lorsque les signaux 20 indiquent une respiration périodique ou des arrêts de respiration.

11. Système selon l'une au moins des revendications précédentes, **caractérisé en ce que** l'unité de commande 3 actionne la source de gaz de respiration 2 pour la spécification d'un écoulement de gaz de respiration prédéfinissable et augmente la pression en fonction de signaux 20 du dispositif de capteur de pression 7 et/ou du dispositif de capteur d'écoulement 8, jusqu'à ce que l'écoulement de gaz de respiration prédéfini soit atteint.

12. Système selon l'une au moins des revendications précédentes, **caractérisé en ce que** l'unité de commande 3 actionne la source de gaz de respiration 2 pour la spécification d'un écoulement de gaz de respiration prédéfinissable, dans lequel le dispositif de capteur d'écoulement 8 surveille l'écoulement de gaz de respiration, dans lequel l'unité de commande vérifie, à l'aide des signaux du dispositif de capteur d'écoulement 8, si l'écoulement de gaz de respiration prédéfini est atteint, dans lequel le dispositif de capteur de pression surveille la pression de gaz de respiration, dans lequel l'unité de commande vérifie, à l'aide des signaux du dispositif de capteur de pression, si une pression de gaz de respiration maximale prédéfinie est atteinte et l'unité de commande n'augmente pas davantage la pression de gaz de respiration maximale lorsque l'écoulement de gaz de respiration prédéfini n'est pas atteint.

13. Système selon l'une au moins des revendications précédentes, **caractérisé en ce que** l'unité de commande active le module de commande d'écoulement 6 et tente ainsi de produire l'écoulement réglé, tout en déterminant également la pression nécessaire à cet effet, si celle-ci atteint un seuil prédéfini pour un laps de temps typiquement d'environ 0,001 à 10 secondes, l'unité de commande passe à la commande de pression 4 et maintient une pression maximale autorisée prédéfinie, également lorsque l'écoulement est inférieur à la spécification réglée, lorsque l'écoulement réglé est de nouveau atteint ou dépassé, pour un laps de temps typiquement d'environ 0,001 à 10 secondes, puis l'unité de commande passe de nouveau à la commande d'écoulement 6.
